# EUROPÄISCHE PATENTSCHRIFT

(11) **EP 1 210 383 B1**
(45) Veröffentlichungstag und Bekanntmachung des Hinweises auf die Patenterteilung: **26.03.2003**
(21) Anmeldenummer: 00956378.4
(22) Anmeldetag: 02.08.2000
(51) Int. Cl.: C08F 290/06, C08F 220/06, A61L 15/60

(54) **POLYMERZUSAMMENSETZUNG UND EIN VERFAHREN ZU DESSEN HERSTELLUNG**
POLYMER COMPOSITION AND A METHOD FOR PRODUCING THE SAME
COMPOSITION POLYMERE ET SON PROCEDE DE PRODUCTION

(30) Priorität: 30.08.1999 DE 19941423
(43) Veröffentlichungstag der Anmeldung: 05.06.2002
(73) Patentinhaber: STOCKHAUSEN GmbH & Co. KG, 47805 Krefeld (DE)
(72) Erfinder: BREHM, Helmut, D-47800 Krefeld (DE); HARTAN, Hans-Georg, D-47625 Kevelaer (DE)
(74) Vertreter: Kahlhöfer, Hermann
(86) Internationale Anmeldenummer: EP0007479
(87) Internationale Veröffentlichungsnummer: WO01016197

(56) Entgegenhaltungen:
- WO-A-97/18889
- DE-A- 19 846 412
- DE-A- 19 846 413
- DE-C- 19 625 143
- US-A- 5 409 771
- US-A- 5 712 316
- US-A- 5 837 789

## Beschreibung

Die Erfindung betrifft ein kontinuierliches Verfahren zur Herstellung hydrophiler, quellfähiger Polymerzusammensetzungen für wäßrige Flüssigkeiten.

Polymerisate, die große Mengen von wäßrigen Flüssigkeiten, insbesondere Körperflüssigkeiten wie Urin aufnehmen, sind als superabsorbierende Polymere bekannt.

Die Herstellung der Polymerisate erfolgt durch radikalische Polymerisation unter vorzugsweiser Verwendung von monoethylenisch ungesättigten Carbonsäuren, wie Acrylsäure und deren Alkalisalze, in wäßriger Lösung oder nach den Verfahren der inversen Suspensions- oder Emulsionspolymerisation, wie sie in US 4,286,082, DE-PS 2706135, US 4,340,706, DE-PS 3713601 und DE-PS 2840010 beschrieben werden.

Durch die Auswahl der Monomerenzusammensetzung, der Vemetzer sowie der Polymerisationsbedingungen und der Verarbeitungsbedingungen für das Polymerisatgel lassen sich Polymerisate mit unterschiedlichen Absorbereigenschaften herstellen. Weitere Variationsmöglichkeiten bietet die Herstellung von Pfropfpolymerisaten, beispielsweise unfer Verwendung chemisch modifizierter Stärke, Cellulose und Polyvinylalkohol nach DE-OS 2512846 und die Nachbehandlung von Polymerisatgelen oder der pulverförmigen Harze durch Nachvemetzung der Oberflächen der Polymerisatpartikel, beispielsweise nach DE 4020780 C1.

Für die Verwendung der Polymerisate im Hygiene- und Sanitärbereich werden Polymerisate erzeugt, deren Neutralisationsgrad etwa zwischen 50 und 80 Mol-%, bezogen auf die polymerisierten, Säuregruppen enthaltenden Monomereinheiten, beträgt, so daß bei der Verwendung hautneutral wirkende Hydrogele gebildet werden.

Im Zuge der technischen Weiterentwicklung der superabsorbierenden Polymere hat sich das Anforderungsprofil an diese Produkte über die Jahre hinweg deutlich verändert. Während in der Entwicklung der Superabsorber zunächst allein das sehr hohe Quellvermögen die Retention bei Kontakt mit Flüssigkeit im Vordergrund stand, hat sich später gezeigt, daß es nicht nur auf die Menge der absorbierten Flüssigkeit ankommt, sondern auch auf die Festigkeit des gequollenen Gels. Retention einerseits und Festigkeit des gequollenen Gels andererseits stellen jedoch gegenläufige Eigenschaften dar, wie bereits aus der US 3,247,171 bekannt ist. Das bedeutet, daß Polymere mit besonders hoher Retention nur eine geringe Festigkeit des gequollenen Gels aufweisen mit der Folge, daß das Gel unter einem angewendeten Druck (z. B. Körperdruck) deformierbar ist und die weitere Flüssigkeitsaufnahme verhindert wird. Diese spezifische Absorptionseigenschaft, die im angelsächsischen Sprachgebrauch "absorption under pressure" (AUP) heißt, wird z. B. in der US 5,314,420 beschrieben.

Im Zuge der gestiegenen Anforderungen an Superabsorber im Hygienebereich hat es sich herausgestellt, daß die ursprüngliche Belastung bei der Messung des AUP von 21 g/cm² (0,3 psi) nicht mehr dem erwünschten Eigenschaftsstandard entspricht, wie er für Inkontinenzprodukte bzw. für Windelkonstruktionen mit niedrigen Fluffgehalten und hohen Mengen Superabsorber erforderlich ist. Demzufolge werden heute Druckbelastungen von 49 g/cm² (0,7 psi) gefordert.

Dem Fachmann sind zwar Methoden bekannt, wie z. B. Produkte mit einer hohen Retention, einer hohen Absorption, niedrigen löslichen Anteilen oder mit einer schnellen Wasseraufnahme jeweils hergestellt werden können. Die gleichzeitige Einstellung aller dieser vier positiven Eigenschaften ist mit den bisher bekannten Rezepturen jedoch nicht möglich. So ist es dem Fachmann bekannt, daß eine Erhöhung der Vernetzerkonzentration Produkte mit niedrigen löslichen Anteilen ergibt, gleichzeitig führt dies aber zu Produkten mit niedriger Retention. Umgekehrt führt die Absenkung der Vernetzerkonzentration zwar zu Produkten mit hohen Retentionen aber ebenso zu hohen löslichen Anteilen.

Die Entwicklung zu immer dünneren Windelkonstruktionen im Hygienebereich geht einher mit einer stärkeren Verdichtung des Absorptionskems unter Zunahme des Superabsorberanteils in der Superabsorber/Zellstoffmischung. Dadurch wird die homogene Flüssigkeitsverteilung im "Absorbent Core" ein immer wichtigeres Kriterium für die vollständige Nutzung der Speicherkapazität der hochquellfähigen Polymere.

Durch die Reduzierung der Zellstoffmenge im Absorptionskern wird die Flüssigkeitsverteilung negativ beeinflußt. Der Einsatz zusätzlicher Hilfsmittel wie spezielle Vliese zur Optimierung der Flüssigkeitsverteilung ist mittlerweile Stand der Technik geworden.

Auch an die hochquellfähigen, flüssigkeitsspeichernden Polymeren werden zusätzliche Anforderungen bezüglich des Flüssigkeitsmanagements gestellt. Die flüssigkeitsspeichernden Polymeren müssen auch in einem Superabsorber/Zellstoffgemisch mit hohem Anteil an hochquellfähigen Polymeren die schnelle Verteilung von Flüssigkeit innerhalb des Superabsorber/Zellstoffgemischs zulassen bzw. unterstützen.

Konventionelle Superabsorber, die häufig gerade bezüglich einer hohen Geschwindigkeit der Flüssigkeitsaufnahme optimiert sind, tendieren dazu, sofort nach dem Kontakt mit wäßriger Flüssigkeit diese Flüssigkeit aufzunehmen und zu speichern. Damit ist eine starke Volumenvergrößerung der Polymerpartikel verbunden.

In der Nähe der Eintrittsstelle von Körperflüssigkeit in den Absorptionskern quellen die absorbierenden Polymeren wegen ihrer hohen Aufnahmegeschwindigkeit gegenüber wäßrigen Flüssigkeiten sehr stark auf, da die Verteilung der Flüssigkeit wegen des geringen Zellstoffanteils im Absorptionskern langsamer erfolgt als die Flüssigkeitsspeicherung durch das hochquellfähige Polymere. Durch diesen Geschwindigkeitsunterschied wird ein sehr großer Teil der Flüssigkeit in unmittelbarer Nähe der Eintrittsstelle absorbiert.

Verstärkend für diesen Effekt kommt hinzu, daß die Quellung eines konventionellen Superabsorbers dergestalt erfolgt, daß unmittelbar nach der Flüssigkeitszugabe eine sehr hohe Absorptionsrate für wäßrige Flüssigkeiten beobachtet wird, verbunden mit einem steilen Anstieg der Absorption. Bereits nach wenigen Minuten hat ein hochquellfähiges Polymeres auf der Basis von vernetztem, teilneutralisiertem Polyacrylat unter den Bedingungen freier Quellung ca. 95 % seiner Absorptionskapazität erreicht. Anschließend nähert sich die aufgenommene Flüssigkeitsmenge asymptotisch ihrem Gleichgewichtswert. Dadurch wird unmittelbar nach der Zugabe der zu absorbierenden Flüssigkeit überproportional viel Flüssigkeit pro Zeiteinheit von den beschriebenen hochquellfähigen Polymeren aufgenommen. Dieses Verhalten ist eine typische Stoffeigenschaft vernetzter Polyacrylate.

Durch die mit der Absorption von Flüssigkeit verbundene Expansion der Polymerpartikel kommt es in dem Bereich des Absorptionskems um die Eintrittsstelle der Körperflüssigkeit zum Verschließen von Zwischenräumen und Poren der SAP-Fluff-Matrix. Da der Flüssigtransport durch Diffusion durch ein gequollenes Hydrogel um Größenordnungen langsamer verläuft als durch Strömung in Zwischenräumen, kommt es zu einer Abdichtung in diesem Bereich. Nachfolgende Mengen an Körperflüssigkeit können nicht mehr in das Absorbent Core eindringen und werden unkontrolliert über die Oberfläche des bereits oberflächlich gesättigten Bereiches bis zu dessen Rand transportiert.

Als Folge davon werden das Rücknäßverhalten und das Leckverhalten (Leakage) des Hygieneprodukts verschlechtert. Zudem sinkt die Speicherkapazität des Absorptionskerns, da tiefer im Absorbent Core eingebettete, hochsaugfähige Polymere durch die Quellung der Partikel an der Oberfläche nicht mehr von weiterer Körperflüssigkeit späterer Dosierungen erreicht werden und dadurch nicht zur Gesamtspeicherkapazität beitragen können.

Um diese Nachteile absorbierender Körper zu beheben, wird in US 5,728,082 vorgeschlagen, zwei unterschiedliche Superabsorber getrennt voneinander im Absorptionskem einzusetzen.

In einer oberen Schicht wird ein hochvemetztes Polymer, d. h. ein hochpermeabler Superabsorber mit geringer Retention und in einer zweiten Schicht ein Polymer mit höherer Retention, d. h. schwächerer Vernetzung installiert.

In EP 0 640 330 A1 wird eine absorbierende Konstruktion beansprucht, die unter anderem zwei getrennte Schichten quellbarer Polymere enthält. die obere Schicht enthält ein Polymer das im gequollenen Zustand eine Permeabilität von mindestens 4 x 10⁻⁷ cm³ sec/g hat, während die untere Polymerschicht eine Absorption unter einer Belastung mit 50 g/cm² von mindestens 15 g/g und einer Quellgeschwindigkeit von mindestens 0,2 g/g·s zeigt.

Aus der DE 195 43 366 A1 sind vernetzte, wasserquellbare Polymerisate mit verbesserter Permeabilität, Retention und Absorption unter Druck bekannt, die unter Verwendung ungesättigten Aminoalkoholen hergestellt werden. Die DE 195 43 368 A1 beschreibt Polymerisate mit verbesserter Absorption unter Druck, bei denen zur Synthese Vernetzermischungen aus Diacrylaten und alkoxylierten Allyletheracrylaten eingesetzt werden. Der DE 196 46 484 A1 sind flüssigkeitsabsorbierende Polymerisate mit verbesserter Permeabilität, Absorption unter Druck und hoher Sauggeschwindigkeit zu entnehmen, die durch die Verwendung einer Kombination aus alkoxylierten Vemetzern und alkoxyliertem Monomer hergestellt werden.

Aufgabe der Erfindung ist es, Polymerzusammensetzungen und ein Verfahren zu deren Herstellung bereitzustellen, die sowohl eine hohe Absorption für wäßrige Flüssigkeiten unter einer Belastung mit 50 g/cm² zeigen und auch eine hohe Permeabilität des gequollenen Gels für wäßrige Flüssigkeiten aufweisen. Ferner sollen die Polymerzusammensetzungen ein Eigenschaftsprofil aufweisen, das zur Zeit nur mit Kombinationen aus getrennt anzuwendenden Polymerisaten bewältigt werden kann.

Die Aufgabe wird erfindungsgemäß durch Bereitstellung einer pulverförmigen, vemetzten Polymerzusammensetzung gelöst, die
a) 55 - 99,9 Gew-% wenigstens eines polymerisierten, ethylenisch ungesättigten, polymerisierbaren, Säuregruppen enthaltenden Monomeren, die mindestens zu 25 Mol-% neutralisiert sind,
b) 0 - 40 Gew-% polymerisierten ungesättigten, mit a) copolymerisierbaren Monomeren,
c) 0,01 - 5,0, vorzugsweise 0,1- 2,0 Gew-% eines oder mehrerer Vernetzungsmittel,
d) 0 - 30 Gew-% eines wasserlöslichen Polymeren,
enthält, wobei die Gewichtsmengen a) bis d) auf die wasserfreie Polymerisatzusammensetzung bezogen sind und die Summe dieser Komponenten immer 100 Gew-% ergeben und die durch kontinuierliche Polymerisation erhältlich ist, bei der mindestens ein die Polymerisation beeinflussender Parameter gemäß einem wiederkehrenden Muster geändert wird.

Als polymerisierbare ungesättigte Säuregruppen enthaltende Monomere a) werden erfindungsgemäß vorzugsweise wasserlösliche monoethylenisch ungesättigte Monound Dicarbonsäuren, besonders bevorzugt Acrylsäure, Methacrylsäure, Ethacrylsäure, Crotonsäure, Sorbinsäure und Maleinsäure, Fumarsäure, Itaconsäure sowie Vinylsulfonsäure, Acrylamido- und/oder Methacrylamido-alkylsulfonsäuren, wie 2-Acrylamido-2-methylpropansulfonsäure, 2-Methacryloyloxiethansulfonsäure, 4-Vinylbenzolsulfonsäure, Allylsulfonsäure, Vinyltoluolsäure, Vinylphosphonsäure und Vinylbenzolphosphonsäure eingesetzt. Die sauren Monomerbestandteile sind mindestens zu 25 Mol%, bevorzugt mindestens zu 50 Mol% und besonders bevorzugt zu 50 bis 80 Mol% neutralisiert.

Eine ganz besonders bevorzugte wasserlösliche ungesättigte Carbonsäure ist die Acrylsäure, die vorzugsweise als einziges Säuregruppen enthaltendes Monomer in den erfindungsgemäßen Polymerisatzusammensetzungen eingesetzt wird und die vorzugsweise zu 50 bis 80 Mol% neutralisiert ist. Ebenfalls bevorzugt beträgt der Anteil anderer ungesättigter Carbonsäuren neben der Acrylsäure im Polymerisat bis zu 50 Gew.%.

Als Monomere b) werden vorzugsweise wasserlösliche monoethylenisch ungesättigte Monomere besonders bevorzugt Acrylamid, Methacrylamid, N-alkylierte (Meth)acrylamide, N-Methylol(meth)acrylamid, N-Vinylamide, N-Vinylformamid, N-Vinylacetamid und N-Vinyl-N-Methylacetamid, N-Vinyl-N-Methylformamid, Vinylpyrrolidon sowie Hydroxylalkyl(meth)acrylate, wie Hydroxyethylacrylat und (Meth)acrylsäureester von Polyethylenglycolmonoallylether und Allylether von Polyethylenglycolen verwendet.

Als Monomere b) werden ebenfalls bevorzugt Monomere mit geringer Löslichkeit in Wasser besonders bevorzugt Acrylsäure- und Methacrylsäureester, wie Ethylacrylat und Methylacrylat, Vinylacetat und Styrol in begrenzter Menge eingesetzt. Der Anteil dieser schwer oder begrenzt wasserlöslichen Monomere beträgt vorzugsweise maximal 10 Gew.%, bezogen auf die Summe aller Monomeren.

Die Monomeren können allein in Lösung oder um Co- und Terpolymerisatzusammensetzungen zu erhalten in Mischung mit anderen Monomeren polymerisiert werden. Der Fachmann erkennt, daß auch mehr als drei Monomeren miteinander polymerisiert werden können.

Die wäßrige Monomerenlösung enthält erfindungsgemäß mindestens einen Vemetzer c) mit einem Anteil von 0,01 - 5,0 Gew-%, vorzugsweise 0,1- 2,0 Gew-%, bezogen auf die wasserfreie, erfindungsgemäße. Als Vemetzer werden vorzugsweise alle Verbindungen verwendet, die mindestens zwei ethylenisch ungesättigte Doppelbindungen oder eine ethylenisch ungesättigte Doppelbindung und eine gegenüber Säuregruppen reaktive funktionelle Gruppe oder mehrere gegenüber Säuregruppen reaktive funktionelle Gruppe enthalten. Beispiele hierfür sind: Methylenbisacrylamid, Acrylate und Methacrylate von Polyolen wie Butandioldiacrylat, Hexandiol-dimethacrylat, Polyethylenglycol-diacrylat und Trimethylolpropantriacrylat und/oder die Acrylate und Methacrylate der oxalkylierten genannten Polyole, wie des oxalkylierten Trimethylolpropans und des oxalkylierten Pentaerythrits. Vernetzer dieses Typs sind unter den Handelsbezeichnungen Sartomer und Craynor (Fa. Crayvalley Kunstharze GmbH, DE-47918 Tönisvorst) bekannt, von denen insbesondere Sartomer 415, Sartomer 454, Sartomer 494, Sartomer 610 und Craynor 435 verwendbar sind, weiterhin Di- und Polyester von Polyolen und oxethylierten Polyolen mit ungesättigten Monocarbonsäuren und/oder Polycarbonsäuren, wie (Meth)acrylsäureestem von 1,2-Propylenglycolpentaerythrit, Glycerin und Polyglycerin sowie Monoester ungesättigter Alkohole und ethoxylierter ungesättigter Alkohole mit ungesättigten Monocarbonsäuren und/oder Monocarbonsäuren wie Allylacrylat und -methacrylat, Monoallylmaleinat, Allylpolyethylenglycoletheracrylat und -methacrylat, Allylitaconat, Allylpolyethylenglycolether-itaconat und Monoallylpolyethylenglycolether-maleinat, weiterhin Diallylacrylamid, Diallylphthalat, Diallyladipat, Triallylcitrat und Trimonoallylpolyethylenglycolethercitrat, weiterhin Allylether von Di- und Polyolen und deren Oxethylate, wie die Diallylether von Ethylenglycol, Diethylenglycol, Polyethylenglycol, die Triallylether von Glycerin, oxethyliertem Glycerin, Trimethylolpropan und oxethyliertem Trimethylolpropan, die Tetraallylether von Pentaerythrit und oxethyliertem Pentaerythrit sowie Tetraallyloxiethan sowie Polyglycidylether, wie z. B. Ethylenglycoldiglycidether und Glyceringlycidylether. Weiterhin Amine und/oder deren Salze und Amide mit mindestens zwei ethylenisch ungesättigten Alkylgruppen, wie Di- und Triallylamin und Tetraallylammoniumchlorid:

Die Monomerenmischung kann weiterhin wasserlösliche Polymere d) mit einem Gehalt von 0 - 30 Gew-%, vorzugsweise 1-5 Gew.-%, bezogen auf die wasserfreie, erfindungsgemäße Polymerzusammensetzung enthalten. Beispiele hierfür sind wasserlösliche Homo- oder Copolymerisate der zuvor genannten Monomeren, wie Polyacrylsäure, teilverseiftes Polyvinylacetat, Polyvinylalkohol, Polyalkylenglykol, Stärke, Stärkederivate, pfropfpolymerisierte Stärke, Cellulose und Cellulosederivate, wie Caboxymethylcellulose, Hydroxymethylcellulose sowie Galaktomannane und dessen oxalkylierte Derivate. Vorzugsweise ist das wasserlösliche Polymere Stärke und/oder Polyvinylalkohol.

Zur Initiierung der radikalischen Polymerisation werden die gebräuchlichen Initiatoren verwendet, z. B. Peroxo- und Azoverbindungen, vorzugsweise in Wasser lösliche und/oder dissoziierende Peroxo- und Azoverbindungen, wie tert. Butylhydroperoxid und 2,2'Azobis'(2-methylpropionamidin)-dihydrochlorid, sowie Redoxsysteme gebildet aus Natrium- und Kaliumperoxomonosulfat, Natrium- und Kaliumperoxidisulfat und Wasserstoffperoxid mit Natrium- und Kaliumsulfit, Natriumund Kaliumformamidinsulfinat und Ascorbinsäure.

Bei Verwendung der Redoxsysteme wird vorzugsweise das Oxydationsmittel vorgelegt und das Reduktionsmittel danach zugegeben. Insbesondere bei kontinuierlicher Polymerisation erfolgt deren Initiierung durch Photokatalyse mit UV-Licht und den bekannten Sensibilisatoren.

Erfindungsgemäß wir bei diesem kontinuierlichen Verfahren mindestens ein die Polymerisation beeinflussender Parameter gemäß einem wiederkehrenden Muster geändert.

Gemäß einem wiederkehrenden Muster im Sinne der Erfindung bedeutet, daß die die Polymerisation beeinflussenden Parameter in einer beliebigen, jedoch sich in regelmäßigen zeitlichen Abständen wiederholenden Weise innerhalb einer sinnvollen Bandbreite, vorzugsweise kontinuierlich, geändert werden.

Vorzugsweise ist das Muster eine Schwingung um einen frei wählbaren Mittelwert. Vorzugsweise ist diese Schwingung harmonisch oder unharmonisch und vorzugsweise ungedämpft.

Vorzugsweise erfolgt die Änderung der Parameter vor dem Beginn der Polymerisation beispielsweise im Zulauf der vorzugsweise wäßrigen Monomerlösung auf den bewegten Träger, wobei vorzugsweise folgende Parameter verändert werden:
a) die Zusammensetzung der Monomerlösung bei der Herstellung von Co- und Terpolymeren, indem die Menge mindestens eines monoethylenisch ungesättigten Monomeren gemäß einem wiederkehrenden Muster verändert wird,
b-1) der Anteil einer oder mehrerer Verbindungen, die mindestens zwei ethylenisch ungesättigte Doppelbindungen enthalten, in dem deren Menge gemäß einem wiederkehrenden Muster verändert dosiert wird,
b-2) der Anteil einer oder mehrerer Verbindungen, die eine ethylenisch ungesättigte Doppelbindung und eine oder mehrere gegenüber Säuregruppen reaktive funktionelle Gruppen enthalten, in dem deren Menge gemäß einem wiederkehrenden Muster verändert dosiert wird,
b-3) der Anteil einer oder mehrerer Verbindungen, die mehrere gegenüber Säuregruppen reaktive funktionelle Gruppen enthalten, in dem deren Menge gemäß einem wiederkehrenden Muster verändert dosiert wird,
c) die Katalysatormenge, indem man die Konzentration des Katalysators oder des Katalysatorsystems gemäß einem wiederkehrenden Muster erhöht und wieder erniedrigt,
d) die Molgewichtsreglermenge, indem man die Menge der Molgewichtsreglerlösung gemäß einem wiederkehrenden Muster erhöht und wieder erniedrigt,
e) der pH-Wert der Monomerlösung bzw. der Neutralisationsgrad, indem man die Alkali-, Endalkali- oder Ammoniakdosierung gemäß einem wiederkehrenden Muster erhöht und wieder erniedrigt,
f) die Pfropfgrundlage, indem man die Menge der Pfropfgrundlage gemäß einem wiederkehrenden Muster erhöht und wieder erniedrigt.

Von diesen Parametern können einer oder mehrere gleichzeitig oder zeitlich versetzt verändert werden.

Vorzugsweise ist das Muster eine Schwingung um einen frei wählbaren Mittelwert. Die Amplitude und Frequenz der Schwingung sind beliebig wählbar. Während die Frequenz, die Zeit in der das Muster der Veränderung eines Polymerisationsparameters einmal durchgeführt wird, durch die Dimensionierung der Anlagenteile bestimmt wird, ist die Amplitude, die Höhe der kontinuierlich durchgeführten Veränderung, entscheidend für die anwendungstechnischen Eigenschaften der Polymerisatzusammensetzungen.

Der Fachmann erkennt, daß die Veränderung eines Parameters zur Veränderung anderer Polymerisationsparameter führen kann. Wird z. B. die Katalysatordosierung im Reaktorzulauf einer Mengenänderung unterworfen, ändert sich laufend die Radikalkonzentration in der Monomerlösung und damit die Polymerisationsgeschwindigkeit - erkennbar am schwingenden Temperaturverlauf - auf dem Polymerisationsband.

Das gebildete Polymergel nach bekanntem Verfahren zerkleinert und bis zu einem Wassergehalt von ca. 10 % bei Temperaturen im Bereich von 100 - 190°C getrocknet.

Anschließend wird das Trockengut auf eine Korngröße im Bereich von 20 - 3000 µm, vorzugsweise 150 - 850 µm zu Polymerisatpulver gemahlen und gesiebt.

Nach der Mahlung bzw. Siebung wird das Polymerisatpulvers vorzugsweise nachvernetzt. Die Nachvernetzung des Polymerisats erfolgt auf der Oberfläche der getrockneten Polymerpartikel mit mindestens einem zwei- oder mehrfach funktionellen, mit Säuregruppen, vorzugsweise Carboxylgruppen, reagierenden Vernetzungsmittel, das vorzugsweise in Form einer wasserhaltigen Lösung aufgebracht wird. Als Nachvemetzungsmittel sind Polyole wie Ethylenglycol, 1,2-Propylenglycol, 1,4-Butandiol, Glycerin, Di- und Polyglycerin, Pentaerythrit, die Oxethylate dieser Polyole sowie deren Ester mit Carbonsäuren oder der Kohlensäure geeignet. Der Zusatz eines Veresterungskatalysators, z. B. p-Toluolsulfonsäure oder Phosphorsäure ist vorteilhaft. Weiter geeignete Vernetzungsmittel sind Di- und Polyglycidylether von Polyolen und Polyehtylenglycolen. Solche Verbindungen sind unter dem Handelsnamen Denacol® (Nagase (Europe) GmbH, Düsseldorf) kommerziell erhältlich. Die Nachvemetzer werden vorzugsweise in Mengen von 0,05 bis 3 Gew.% bezogen auf die Polymerzusammensetzung eingesetzt.

Die Nachvernetzung wird vorzugsweise bei Temperaturen im Bereich von 150 - 250 °C, besonders bevorzugt 150 - 200 °C in einem Mischaggregat, vorzugsweise in einem Naramischer vorgenommen.

Die erfindungsgemäße Polymerisatzusammensetzung läßt sich besonders vorteilhaft als Absorptionsmittel für Wasser und wässrige Flüssigkeiten, als Absorptionsmittel in Konstruktionen zur Aufnahme von Körperflüssigkeiten, als absorbierende Komponente in strom- oder lichtleitenden Kabeln bzw. Verpackungsmaterialien, als Bodenverbesserungsmittel, bei der Pflanzenzucht, als Absorptionsmittel für Wasser und wässrige Flüssigkeiten in vorzugsweise geschäumten Flächengebilden und als Trägersubstanz für Düngemittel pder andere Wirkstoffe, die wieder über einen längeren Zeitraum abgegeben werden, einsetzen. Diese Verwendungen sind deshalb ebenfalls Gegenstand der vorliegenden Erfindung.

Ein weiter Gegenstand der vorliegenden Erfindung ist ein kontinuierliches Verfahren zur Herstellung der erfindungsgemäßen, pulverförmigen, vemetzten, wäßrige oder seröse Flüssigkeiten sowie Blut absorbierenden Polymerisatzusammensetzungen, enthaltend:
a) 55 - 99,9 Gew-% wenigstens eines polymerisierten, ethylenisch ungesättigten, polymerisierbaren, Säuregruppen enthaltenden Monomeren, die mindestens zu 25 Mol-% neutralisiert sind,
b) 0 - 40 Gew-% polymerisierten ungesättigten, mit a) copolymerisierbaren Monomeren,
c) 0,01 - 5,0, vorzugsweise 0,1- 2,0 Gew-% eines oder mehrerer Vernetzungsmittel,
d) 0 - 30 Gew-% eines wasserlöslichen Polymeren,
wobei die Gewichtsmengen a) bis d) auf die wasserfreie Polymerisatzusammensetzung bezogen sind und die Summe dieser Komponenten immer 100 Gew-% ergeben. Bei dem erfindungsgemäßen Verfahren wird mindestens ein die Polymerisation beeinflussender Parameter gemäß einem wiederkehrenden Muster geändert.

Bezüglich der Komponenten a) bis d) wird auf die Offenbarung betreffend die erfindungsgemäße Polymerisatzusammensetzung verwiesen.

Die Polymerisation erfolgt nach jedem dem Fachmann bekannten Verfahren. Vorzugsweise ist die Polymerisation eine Lösungspolymerisation. Vorzugsweise wird die kontinuierliche Lösungspolymerisation auf einem bewegten Träger durchgeführt, wie er z. B. in der EP 0296331 B1 oder in der EP 0228638 B1 gelehrt wird, die hiermit als Referenz eingeführt werden und somit als Teil der Offenbarung gelten. In diesen Patentschriften ist der bewegte Träger ein Förderband, auf das eine wäßrige, vom Sauerstoff befreite Monomerlösung dosiert wird, die nach Zusatz von Katalysatoren unter Freiwerden der Polymerisationswärme zu einem festen Gel polymerisiert.

Bei dem erfindungsgemäßen, kontinuierlichen Verfahren wird mindestens ein die Polymerisation beeinflussender Parameter gemäß einem wiederkehrenden Muster geändert.

Gemäß einem wiederkehrenden Muster im Sinne der Erfindung bedeutet, daß die die Polymerisation beeinflussenden Parameter in einer beliebigen, jedoch sich in regelmäßigen zeitlichen Abständen wiederholenden Weise innerhalb einer sinnvollen Bandbreite, vorzugsweise kontinuierlich, geändert werden.

Vorzugsweise ist das Muster eine Schwingung um einen frei wählbaren Mittelwert. Vorzugsweise ist diese Schwingung harmonisch oder unharmonisch und vorzugsweise ungedämpft.

Vorzugsweise erfolgt die Änderung der Parameter vor dem Beginn der Polymerisation beispielsweise im Zulauf der vorzugsweise wäßrigen Monomerlösung auf den bewegten Träger, wobei vorzugsweise folgende Parameter verändert werden:
a) die Zusammensetzung der Monomerlösung bei der Herstellung von Co- und Terpolymeren, indem man die Menge mindestens eines monoethylenisch ungesättigten Monomeren gemäß einem wiederkehrenden Muster verändert,
b-1) der Anteil einer oder mehrerer Verbindungen, die mindestens zwei ethylenisch ungesättigte Doppelbindungen enthalten, in dem deren Menge gemäß einem wiederkehrenden Muster verändert dosiert wird,
b-2) der Anteil einer oder mehrerer Verbindungen, die eine ethylenisch ungesättigte Doppelbindung und eine oder mehrere gegenüber Säuregruppen reaktive funktionelle Gruppen enthalten, in dem deren Menge gemäß einem wiederkehrenden Muster verändert dosiert wird,
b-3) der Anteil einer oder mehrerer Verbindungen, die mehrere gegenüber Säuregruppen reaktive funktionelle Gruppen enthalten, in dem deren Menge gemäß einem wiederkehrenden Muster verändert dosiert wird,
c) die Katalysatormenge, indem man die Konzentration des Katalysators oder des Katalysatorsystems gemäß einem wiederkehrenden Muster erhöht und wieder erniedrigt,
d) die Molgewichtsreglermenge, indem man die Menge der Molgewichtsreglerlösung gemäß einem wiederkehrenden Muster erhöht und wieder erniedrigt,
e) der pH-Wert der Monomerlösung bzw. der Neutralisationsgrad, indem man die Alkali-, Endalkali- oder Ammoniakdosierung gemäß einem wiederkehrenden Muster erhöht und wieder erniedrigt,
f) die Pfropfgrundlage, indem man die Menge der Pfropfgrundlage gemäß einem wiederkehrenden Muster erhöht und wieder erniedrigt.

Von diesen Parametern können einer oder mehrere gleichzeitig oder zeitlich versetzt verändert werden.

Vorzugsweise ist das Muster eine Schwingung um einen frei wählbaren Mittelwert. Die Amplitude und Frequenz der Schwingung sind beliebig wählbar. Während die Frequenz, die Zeit in der das Muster der Veränderung eines Polymerisationsparameters einmal durchgeführt wird, durch die Dimensionierung der Anlagenteile bestimmt wird, ist die Amplitude, die Höhe der kontinuierlich durchgeführten Veränderung, entscheidend für die anwendungstechnischen Eigenschaften der Polymerisatzusammensetzungen.

Der Fachmann erkennt, daß die Veränderung eines Parameters zur Veränderung anderer Polymerisationsparameter führen kann. Wird z. B. die Katalysatordosierung im Reaktorzulauf einer Mengenänderung unterworfen, ändert sich laufend die Radikalkonzentration in der Monomerlösung und damit die Polymerisationsgeschwindigkeit - erkennbar am schwingenden Temperaturverlauf - auf dem Polymerisationsband.

Das gebildete Polymergel wird nach bekanntem Verfahren zerkleinert und bis zu einem Wassergehalt von ca. 10 % bei Temperaturen im Bereich von 100 - 190 °C getrocknet.

Anschließend wird das Trockengut auf eine Korngröße im Bereich von 20 - 3000 µm, vorzugsweise 150 - 850 µm zu Polymerisatpulver gemahlen und gesiebt.

Nach der Mahlung bzw. Siebung wird das Polymerisatpulvers vorzugsweise nachvemetzt. Die Nachvernetzung des Polymerisats erfolgt auf der Oberfläche der getrockneten Polymerpartikel mit mindestens einem zwei- oder mehrfach funktionellen, mit Säuregruppen, vorzugsweise Carboxylgruppen, reagierenden Vernetzungsmittel, das vorzugsweise in Form einer wasserhaltigen Lösung aufgebracht wird. Als Nachvernetzungsmittel sind Polyole wie Ethylenglycol, 1,2-Propylenglycol, 1,4-Butandiol, Glycerin, Di- und Polyglycerin, Pentaerythrit, die Oxethylate dieser Polyole sowie deren Ester mit Carbonsäuren oder der Kohlensäure geeignet. Der Zusatz eines Veresterungskatalysators, z. B. p-Toluolsulfonsäure oder Phosphorsäure ist vorteilhaft. Weiter geeignete Vernetzungsmittel sind Di- und Polyglycidylether von Polyolen und Polyehtylenglycolen. Solche Verbindungen sind unter dem Handelsnamen Denacol ® (Nagase (Europe) GmbH, Düsseldorf) kommerziell erhältlich. Die Nachvernetzer werden vorzugsweise in Mengen von 0,05 bis 3 Gew.% bezogen auf die Polymerzusammensetzung eingesetzt.

Die Nachvernetzung wird vorzugsweise bei Temperaturen im Bereich von 150 - 250 °C, besonders bevorzugt 150 - 200 °C in einem Mischaggregat, vorzugsweise in einem Naramischer vorgenommen.

Zur Durchführung des erfindungsgemäßen Verfahrens wird z.B. die in der EP 0 296 331 B1, Beispiel 4 und Fig. 2 beschriebene kontinuierliche Polymerisation dahingehend verändert, daß über die Dosierventile 28 und 30 der Massedurchfluß der Katalysatorlösungen nach einem vorgewählten Muster über eine Regelstrecke in regelmäßigen zeitlichen Abständen wiederkehrend verändert werden. Die oben genannte Patentschrift wird hiermit als Referenz eingeführt und gilt somit als Teil der Offenbarung.

In einer bevorzugten Ausführungsform des erfindungsgemäßen Verfahrens wird in den konstanten Strom einer mit Stickstoff gespülten, teilneutralisierten Lösung von Acrylsäure, die zu 70 Mol-% als Natriumacrylat vorliegt, eine konstante Menge eines Katalysatorsystems dosiert. Aus einer weiteren Vorlage wird eine Lösung des Vemetzers Triallylamin in Methacrylsäure mit sich ständig in Form einer sinusförmigen Schwingung ändernden Menge dosiert. Je nach Konzentration und Menge der Vemetzerlösung ändert sich periodisch nicht nur die Vemetzungsdichte und der Neutralisationsgrad des Polymeren, sondern auch die Monomerkonzentration auf dem Polymeristionsband, was zu einem - aufgrund der exothermen Reaktion - sich ständig ändernden Temperaturverlauf im Polymergel führt. Da am Trocknerende die Pfropfenströmung der Anlage noch weitgehend erhalten ist, zeigen im Abstand von 5 Min. gezogenen Proben deutlich die sich ändernde Vernetzungsdichte, erkennbar an der sich ändernden Aufnahmekapazität von physiologischer Kochsalzlösung.

In einer weiteren bevorzugten Ausführungsform des erfindungsgemäßen Verfahrens wird in den konstanten Zulaufstrom einer mit Stickstoff gespülten, zu 60 Mol-% mit Natronlauge neutralisierten Acrylsäurelösung, die bereits einen Vemetzer z. B. Allylpolyethylenglykoletheracrylat enthält, nach kontinuierlicher Zugabe der Katalysatoren, zusätzlich eine sich periodisch ändernde Menge einer wäßrigen Lösung von z. B. PEG(Polyethylenglykol)-400-diacrylat dosiert. Die am Trocknerende gezogenen Proben zeigen eine sich in Form einer Schwingung ändernde Flüssigkeitsabsorption. Nach dem Durchgang des getrockneten Polymeren durch Anlagenteile mit starker Rückvermischung wie Mühlenkreislauf, Siebung und Silomischer liegt ein Haufwerk vor, das bei der Prüfung nahezu homogen ist.

In einer anderen bevorzugten Ausführungsform des erfindungsgemäßen Verfahrens wird in den konstanten Zulaufstrom einer mit Stickstoff gespülten, zu 70 Mol-% mit Natronlauge neutralisierten Acrylsäurelösung, die bereits einen Vernetzer z. B. Allylpolyethylenglykoletheracrylat enthält, nach kontinuierlicher Zugabe der Katalysatoren, zusätzlich eine sich periodisch ändernde Menge einer wäßrigen Lösung eines nichtionogenen Monomeren z. B. Methoxy-PEG(Polyethylenglykol)-750-methacrylat dosiert.

Anstelle des nichtionogenen Monomeren kann auch die Lösung einer Pfropfgrundlage wie z. B. Polyvinylalkohol dosiert und die Menge gemäß einem wiederkehrenden Muster geändert werden.

Enthält die Lösung der Pfropfgrundlage einen Regler wie z. B. Ameisensäure, Essigsäure oder Isopropanol, so ändert sich die Menge bezogen auf das Monomere ebenfalls periodisch. Sie ist aber dann am höchsten, wenn die Pfropfgrundlage ihre höchste Konzentration in der polymerisierenden Lösung erreicht.

Dem Fachmann ist bekannt, daß sich Polymergele mit unterschiedlicher Vernetzungsdichte, unterschiedlichem Neutralisationsgrad, unterschiedlicher Temperatur usw. nach der Polymerisation bezüglich Schneid-, Trocken- und Mahlbarkeit deutlich unterscheiden können.

Deshalb wird vorgeschlagen, die Polymerisation auf zwei parallel betriebenen Polymerisationsbändem durchzuführen. Erfolgt die Änderung der die Polymerisation beeinflussenden Parameter jeweils phasenverschoben, d. h. wenn z. B. die Vernetzerkonzentration auf dem einen Band dem Maximum zustrebt, nähert sich die auf dem anderen Band dem Minimum, können die Gelstränge gemeinsam unter konstanten Bedingungen zerkleinert, getrocknet und gemahlen werden.

Die erfindungsgemäßen Polymerzusammensetzungen haben den Vorteil, daß sie einfach auf bestehenden Anlagen herzustellen sind und daß sie eine große Bandbreite superabsorbierender Polymere abdecken.

Dies gilt insbesondere für Polymerzusammensetzungen, deren Oberfläche nachvernetzt wurde

Durch die Nachvernetzung in der Oberflächenschicht der Polymerpartikel entsteht eine Polymerzusammensetzung, die eine große Bandbreite nachvernetzter superabsorbierender Polymere abdeckt.

Beispielsweise kann eine erfindungsgemäße Polymerzusammensetzung gleitende Anteile hoher Retention mit niedriger Vernetzung neben gleitenden Anteilen niedriger Retention mit hoher Absorption unter Belastung und hoher Gelpermeabilität enthalten. Die Eigenschaften derartiger Polymerzusammensetzungen sind in der Anwendung den Mischungen, wie sie im Stand der Technik beschrieben werden, überlegen.

Im Folgenden wird die Erfindung anhand von Beispielen erklärt, die jedoch den allgemeinen Erfindungsgedanken nicht einschränken.

### Testmethoden

### Teebeutel Retention (TB)

Dieser Test wird entsprechend den Angaben in EP 0640330 A1 unter "Teabag Retention Capacity Test" durchgeführt.

### Absorption unter Betastung (AUB)

Dieser Test wird entsprechend den Angaben in EP 0640330 A1 unter "Absorption Against Pressure Test" bei Belastungen mit 20 g/cm² (0,3 psi) und 50 g/cm² (0,7 psi) durchgeführt.

### Gelpermeabilität (GP)

Diese Test wird entsprechend den Angaben in EP 0640330 A1 unter "Gel Layer Permeability Test" durchgeführt.

### Passing Rate (PR)

Der Test wird entsprechend den Angaben in EP 594 009 B1 unter "Method for measuring passing rate of physiological saline" durchgeführt.

### Beispiele

### Abkürzungen:

- Methoxipolyethylenglycol(17EO)-methacrylat =: MPEG-MAC
- Polyethylenglycol(10EO)-allyletheracrylat =: PEGMAE-AC
- Natriumperoxidisulfat =: NAPS
- 2,2'-Azobis(2-methylpropionamidin)-dihydrochlorid =: ABAH

### Beispiel 1

| Ansatz: | |
|---|---|
| 4000 kg | Wasser |
| 2030 kg | Natronlauge 50%ig |
| 2610 kg | Acrylsäure |
| 23,5 kg | PEGMAE-AC |
| 8.663,5 kg | Monomerlösung |

400 kg/h dieser Monomerlösung werden in einem Wärmetauscher auf 1°C abgekühlt und in einem mit 3 m³/h Stickstoff durchströmten Stripper vom gelösten Sauerstoff bis auf einen Restgehalt von 0,9 ppm befreit. Mit diesem Zulauf 1 werden vor der Aufgabe auf das Polymersationsband die folgenden Lösungsmengen vermischt:

| | | | |
|---|---|---|---|
| Zulauf 2 | 8,8 l/h | NAPS-Lösung | 0,75%ig |
| Zulauf 3 | 8,8 l/h | ABAH-Lösung | 0,5%ig |
| Zulauf 4 | 8,8 l/h | Wasserstoffperoxidlösung | 0,5%ig |
| Zulauf 5 | 8,8 l/h | Ascorbinsäurelösung | 0,075%ig |
| Zulauf 6 | Eine Lösung aus 15,66 kg Polyethylenglycol-300-diacrylat und 162 kg Wasser, deren Dosierungsrate in einer Zeit von 30 min. von 5 kg/h gleichmäßig auf 10 kg/h ansteigend und gleichmäßig abnehmend auf 5 kg/h geändert wird. Dieses Muster der Mengenänderung wird während der gesamten Versuchsdauer beibehalten. | | |

Nach einer Verweilzeit von 40 min. wird das feste, noch heiße Polymergel mit einem Fleischwolf zerkleinert und auf einem Bandtrockner in Zone 1 und 2 mit 160 °C, Zone 3 mit 140 °C und Zone 4 und 5 mit 130 °C Zulufttemperatur getrocknet. Die am Ende des Trockners im Abstand von 5 min. gezogenen Proben zeigen folgende Retentionen:

| | | | | | | | |
|---|---|---|---|---|---|---|---|
| Zeit [min] | 0 | 5 | 10 | 15 | 20 | 25 | 30 |
| TB [g/g] | 27 | 27,5 | 28 | 29 | 28,5 | 27 | 26,5 |

Die gemahlene und auf 150 bis 850 µm abgesiebte Siloprobe hat folgende Kornverteilung:

| Siebanalyse | | | | | |
|---|---|---|---|---|---|
| Maschenweite [µm] | 850 | 600 | 300 | 150 | 45 |
| % auf Sieb | 0,1 | 3,9 | 67,3 | 28,6 | 0,1 |

### Beispiel 1a und 1b

Die Polymerzusammensetzung aus Beispiel 1 wird kontinuierlich mit einer pneumatischen Förderung in die Pulvervorlage einer Dosierbandwaage gefördert und mit 80 kg/h Durchsatz in einem vertikalen Rohrmischer kontinuierlich über eine 2-Stoffdüse mit 2 % einer 33,5%igen Ethylencarbonatlösung in Wasser vermischt. Die Mischung durchfließt in ca. 20 Minuten einen mit Dampf beheizten Paddeltrockner. Der Dampf wies die in der Tabelle angegebenen Temperaturen auf. Nach der Abkühlung des Polymerpulvers auf einem Schwingkühler erfolgt nach der pneumatischen Förderung eine Schutzsiebung bei 1.000 µm und eine weitere Förderung zum Silo. Die Siloproben zeigen folgende Kenndaten:

| **Beispiel** | **Dampftemp.** | **TB** | **AUB** | | **GP** |
|---|---|---|---|---|---|
| | | | 20 g/cm² | 50 g/cm² | |
| | [°C] | [g/g] | [g/g] | [g/g] | [cm³ s/g] |
| 1a | 192 | 24,5 | 27 | 24 | 86×10⁻⁷ |
| 1b | 190 | 26 | 27,5 | 23,5 | 67×10⁻⁷ |

### Beispiel 2

| Ansatz: | |
|---|---|
| 4000 kg | Wasser |
| 2030 kg | Natronlauge 50%ig |
| 2610 kg | Acrylsäure |
| 105 kg | MPEG-MAC |
| 15,7 kg | PEGMAE-AC |
| 8.760,7 kg | Monomerlösung |

Diese Monomerlösung, die wie in Beispiel 1 behandelt wird, bildet mit 400 kg/h den Zulauf 1, der wie in Beispiel 1 mit den dort angegebenen Katalysatorlösungen 2 bis 5 vor dem Polymerisationsband vermischt wird.

Zulauf 6: Eine Lösung von 15 kg Polyethylenglycol-400-dimethacrylat in 200 kg Wasser, deren Dosierungsrate in einer Zeit von 60 min. von 5 kg/h gleichmäßig auf 15 kg/h ansteigend und gleichmäßig abnehmend auf 5 kg/h geändert wird. Dieses Muster der Mengenänderung wird während der gesamten Versuchsdauer beibehalten.

Nach einer Verweilzeit von 40 min. wird das Polymergel wie in Beispiel 1 zerkleinert und getrocknet. Die am Ende des Trocknens im Abstand von 5 Minuten gezogenen Proben haben folgende Retention:

| | | | | | | | | | | | | | |
|---|---|---|---|---|---|---|---|---|---|---|---|---|---|
| Zeit [min] | 0 | 5 | 10 | 15 | 20 | 25 | 30 | 35 | 40 | 45 | 50 | 55 | 60 |
| TB [g/g] | 35,6 | 36,4 | 37,2 | 36,3 | 35,2 | 34,9 | 34,2 | 33,2 | 33,1 | 31,9 | 32,5 | 33,8 | 35 |

Die gemahlene und auf 150 bis 850 µm abgesiebte Siloprobe hat folgende Kornverteilung:

| Siebanalyse | | | | | |
|---|---|---|---|---|---|
| Maschenweite [µm] | 850 | 600 | 300 | 150 | 45 |
| % auf Sieb | 0,2 | 25,7 | 60,9 | 13,1 | 0,1 |

### Beispiele 2a und 2b

Die Prepolymerzusammensetzung wird wie in den Beispielen 1a und 1b thermisch nachbehandelt.

| **Nachbehandlung und Kenndaten:** | | | | | | |
|---|---|---|---|---|---|---|
| **Beispiel** | **EC* / Wasser** | **Dampftemp** | **TB** | **AUB** | | **GP** |
| | | | | 20 g/cm² | 50 g/cm² | |
| | [%[ / [%] | [°C] | [g/g] | [g/g] | [g/g] | [cm³ s/g] |
| 2a | 0,5 / 1,0 | 190 | 29,5 | 31 | 26 | 22x10⁻⁷ |
| 2b | 1,0 / 2,5 | 194 | 28 | 28,5 | 23 | 81x10⁻⁷ |

| | | | | | | |
|---|---|---|---|---|---|---|
| * Ethylencarbonat | | | | | | |

### Vergleichsbeispiel 1

Beispiel 2 und 2 b werden wiederholt, aber mit der Maßnahme, daß der Zulauf 6 in Beispiel 2 über die Zeit konstant bei 10 kg/h gehalten wird. TB: 34 g/g

In der nachfolgenden Tabelle sind die Kenndaten von Beispiel 2b und dem Vergleichsbeispiel 1 ergänzt um die Angabe der Durchflußgeschwindigkeit (passing rate = PR) für physiologische Kochsalzlösung durch das vorgequollene Polymer. Die Durchführung dieser Prüfmethode erfolgt nach EP 0594009 B1.

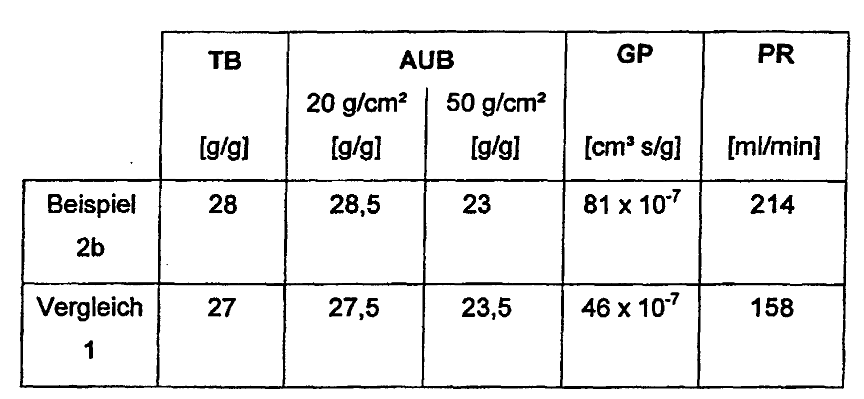

Das erfindungsgemäße Polymerisat weist eine deutlich höhere Permeabilität auf.

## Patentansprüche

1. Pulverförmige, vernetzte Polymerisatzusammensetzung zur Absorption von wässrigen oder serösen Flüssigkeiten sowie Blut enthaltend:
a) 55 - 99,9 Gew-% wenigstens eines polymerisierten, ethylenisch ungesättigten, polymerisierbaren, Säuregruppen enthaltenden Monomeren, die mindestens zu 25 Mol-% neutralisiert sind,
b) 0 - 40 Gew-% polymerisierten ungesättigten, mit a) copolymerisierbaren Monomeren,
c) 0,01 - 5,0, vorzugsweise 0,1 - 2,0 Gew-% eines oder mehrerer Vernetzungsmittel,
d) 0 - 30 Gew-% eines wasserlöslichen Polymeren,
wobei die Gewichtsmengen a) bis d) auf die wasserfreie Polymerisatzusammensetzung bezogen sind und die Summe dieser Komponenten immer 100 Gew-% ergeben, erhältlich durch kontinuierliche Polymerisation, bei der mindestens ein die Polymerisation beeinflussender Parameter gemäß einem wiederkehrenden Muster geändert wird.

2. Polymerisatzusammensetzungen nach Anspruch 1, **dadurch gekennzeichnet, dass** das Muster eine Schwingung um einen frei wählbaren Mittelwert ist.

3. Polymerisatzusammensetzungen nach Anspruch 2, **dadurch gekennzeichnet, dass** die Schwingung harmonisch oder unharmonisch und vorzugsweise ungedämpft ist.

4. Polymerisatzusammensetzungen nach einem der Ansprüche 1 bis 3, **dadurch gekennzeichnet, dass** mindestens einer der folgenden Parameter:
- die Konzentration des/der Vernetzungsmittel,
- die Katalysatormenge,
- die Molgewichtsreglermenge,
- die Zusammensetzung der Monomerenlösung und/oder
- der pH-Wert der Monomerlösung
geändert wird.

5. Polymerisatzusammensetzungen nach cincm dcr Ansprüche 1 bis 4, **dadurch gekennzeichnet, dass** die Säuregruppen enthaltenden Monomeren Acrylsäure, Methacrylsäure und/oder 2-Acrylamido-2-methylpropansulfonsäure sind.

6. Verfahren zur kontinuierlichen Herstellung pulverförmiger, vernetzter, wässrige oder seröse Flüssigkeiten sowie Blut absorbierender Polymerisatzusammensetzungen, enthaltend:
a) 55 - 99,9 Gew-% wenigstens eines polymerisierten, ethylenisch ungesättigten, polymerisierbaren, Säuregruppen enthaltenden Monomeren, die mindestens zu 25 Mol-% neutralisiert sind,
b) 0 - 40 Gew-% polymerisierten ungesättigten, mit a) copolymerisierbaren Monomeren,
c) 0,01 - 5,0, vorzugsweise 0,1 - 2,0 Gew-% eines oder mehrerer Vernetzungsmittel,
d) 0 - 30 Gew-% eines wasserlöslichen Polymeren,
wobei die Gewichtsmengen a) bis d) auf die wasserfreie Polymerisatzusammensetzung bezogen sind, die Summe dieser Komponenten immer 100 Gew-% ergeben und die Monomerenlösung zu einem Gel polymerisiert und das Gel getrocknet und zerkleinert wird, **dadurch gekennzeichnet, dass** mindestens ein die Polymerisation beeinflussender Parameter gemäß einem wiederkehrenden Muster geändert wird.

7. Verfahren nach Anspruch 6, **dadurch gekennzeichnet, dass** mindestens einer der folgenden Parameter:
- die Konzentration des/der Monomeren,
- die Katalysatormenge,
- die Molgewichtsreglermenge,
- der ph-Wert der Monomerlösung und/oder
- die Zusammensetzung der eingesetzten Monomeren
geändert wird.

8. Verfahren nach einem der Ansprüche 6 oder 7, **dadurch gekennzeichnet, dass** die Polymerisation auf einem bewegten Träger erfolgt.

9. Verfahren nach einem der Ansprüche 6 bis 8, **dadurch gekennzeichnet, dass** die Polymerisatzusammensetzung nach der Trocknung pulverisiert wird und das pulverförmige Polymerisat mit 0,05 bis 3 Gew-% einer Verbindung, die mit mindestens zwei Carboxylgruppen reagieren kann, vermischt und auf 150 - 250°C erhitzt und dabei oberflächenvernetzt werden.

10. Verwendung der Polymerisatzusammensetzung nach einem der Ansprüche 1-5 als Absorptionsmittel für Wasser und wässrige Flüssigkeiten.

11. Verwendung der Polymerisatzusammensetzung nach einem der Ansprüche 1-5 als Absorptionsmittel in Konstruktionen zur Aufnahme von Körperflüssigkeiten.

12. Verwendung der Polymerisatzusammensetzung nach einem der Ansprüche 1-5 als Wasser und wässrige Flüssigkeiten absorbierende Komponente in strom- oder lichtleitenden Kabeln, als Komponente in Verpackungsmaterialien, als Bodenverbesserungsmittel und bei der Pflanzenzucht.

13. Verwendung der Polymerisatzusammensetzung nach einem der Ansprüche 1-5 als Absorptionsmittel für Wasser und wässrige Flüssigkeiten in vorzugsweise geschäumten Flächengebilden.

14. Verwendung der Polymerisatzusammensetzung nach einem der Ansprüche 1-5 als Trägersubstanz für Düngemittel oder andere Wirkstoffe, die wieder über einen längeren Zeitraum abgegeben werden.

## Claims

1. Powdered, crosslinked polymer composition for absorbing aqueous or serous liquids as well as blood, containing:
a) 55 - 99,9 wt.-% of at least one polymerized, ethylenically unsaturated, polymerizable monomer, which contains acid groups neutralized to at least 25 mole-%,
b) 0 - 40 wt.-% of polymerized, unsaturated monomers, copolymerizable with a),
c) 0,01 - 5,0 wt.-%, preferably 0,1 - 2,0 wt.-% of one or more crosslinking agents,
d) 0 - 30 wt.-% of a water-soluble polymer,
the weight amounts a) through d) being based on the anhydrous polymer composition, and the sum of these components always being 100 wt.-%, which compositions can be obtained by continuous polymerization, wherein at least one parameter biasing the polymerization is varied according to a recurring pattern.

2. The polymer compositions according to claim 1, **characterized in that** the pattern is an oscillation about a mean value, which can be selected at random.

3. The polymer compositions according to claim 2, **characterized in that** de oscillation is harmonic or inharmonic and preferably undamped.

4. The polymer compositions according to any of claims 1 to 3, **characterized by** varying at least one of the following parameters:
- the concentration of the crosslinking agent(s)
- the amount of catalyst,
- the amount of molecular weight modifier,
- the composition of the monomer solution and/or
- the pH value of the monomer solution.

5. The polymer compositions according to any of claims 1 to 4, **characterized in that** the monomers containing acid groups are acrylic acid, methacrylic acid and/or 2-acrylamido-2-methylpropansulfonic acid.

6. A process for the continuous production of powdered, crosslinked polymer compositions absorbing aqueous or serous fluids, as well as blood, containing:
a) 55 - 99,9 wt.-% of at least one polymerized, ethylenically unsaturated, polymerizable monomer, which contains acid groups neutralized to at least 25 mole-%,
b) 0 - 40 wt.-% of polymerized, unsaturated monomers, copolymerizable with a),
c) 0,01 - 5,0 wt.-%, preferably 0,1 - 2,0 wt.-% of one or more crosslinking agents,
d) 0 - 30 wt.-% of a water-soluble polymer,
the weight amounts a) through d) being based on the anhydrous polymer composition, the sum of these components always being 100 wt.-%, and the monomer solution being polymerized to form a gel, and said gel being dried and crushed, **characterized in that** at least one parameter biasing the polymerization is varied according to a recurring pattern.

7. The process according to claim 6, **characterized by** varying at least one of the following parameters:
- the concentration of the monomer(s),
- the amount of the catalyst,
- the amount of molecular weight modifier,
- the pH value of the monomer solution and/or
- the composition of the monomer solution used.

8. The process according to any of claims 6 or 7, **characterized in that** the polymerization is effected on a moving support.

9. The process according to any of claims 6 to 8, **characterized in that** the polymer composition is pulverized after drying and the powdered polymer is mixed with 0,05 - 3 wt.-% of a compound capable of reacting with at least to carboxyl groups and heated to 150-250°C and thereby undergoing surface crosslinking.

10. Use of the polymer composition according to any of claims 1 to 5 as absorbent for water and aqueous liquids.

11. Use of the polymer composition according to any of claims 1 to 5 as an absorbent in constructions used to absorb body liquids.

12. Use of the polymer composition according to any of claims 1 to 5 as a component in electroconductive or light-conducting cables, which absorbs water and aqueous liquids, as a component in packaging materials, as soil improver, and in plant breeding.

13. Use of the polymer composition according to any of claims 1 to 5, as an absorbent for water and aqueous liquids in preferably foamed sheet materials.

14. Use of the polymer composition according to any of claims 1 to 5 as carrier substance for fertilizers or other active substances released over a prolonged period of time.

## Revendications

1. Composition de polymères réticulée en poudre pour l'absorption de liquides aqueux ou séreux ainsi que de sang, comprenant :
a) 55 - 99,9 % en poids d'au moins un monomère à insaturation éthylénique polymérisé contenant des groupes acides, polymérisable, neutralisé à au moins 25 % en mol,
b) 0 - 40 % en poids de monomères insaturés polymérisés co-polymérisables avec a),
c) 0,01 - 5,0 de préférence 0,1 - 2,0 % en poids d'un ou de plusieurs agents de réticulation,
d) 0 - 30 % en poids d'un polymère soluble dans l'eau,
les quantités de poids a) à d) se référant à la composition de polymères sans eau, et la somme de ces composants étant toujours 100 % en poids, pouvant être obtenue par polymérisation continue où au moins un paramètre influençant la polymérisation est modifié selon un modèle périodique.

2. Composition de polymères selon la revendication 1, **caractérisée en ce que** le modèle est une oscillation autour d'une valeur moyenne quelconque.

3. Composition de polymères selon la revendication 2, **caractérisée en ce que** cette oscillation est harmonique ou non et de préférence sans amortissement.

4. Composition de polymères selon l'une des revendications 1 à 3, **caractérisée en ce qu'**au moins l'un des paramètres suivants est modifié :
- la concentration du/des agent(s) de réticulation(s)
- la quantité de catalyseur
- la quantité du régulateur du poids molaire
- la composition de la solution de monomères et/ou
- la valeur du pH de la solution de monomères.

5. Composition de polymères selon l'une des revendications 1 à 4, **caractérisée en ce que** les monomères contenant des groupes acides sont l'acide acrylique, l'acide méthacrylique et/ou l'acide 2-acrylamido-2-méthylpropylsulfonique.

6. Procédé continu pour la production de compositions de polymères en poudre réticulées absorbant des liquides aqueux ou séreux ainsi que du sang, comprenant :
a) 55 - 99,9 % en poids d'au moins un monomère à insaturation éthylénique polymérisé contenant des groupes acides, polymérisable, neutralisé à au moins 25 en mol,
b) 0 - 40 % en poids de monomères insaturés polymérisés co-polymérisables avec a),
c) 0,01 - 5,0 de préférence 0,1 - 2,0 % en poids d'un ou de plusieurs agents de réticulation,
d) 0 - 30 % en poids d'un polymère soluble dans l'eau,
les quantités de poids a) à d) se référant à la composition de polymères sans eau, et la somme de ces composants étant toujours 100 % en poids, et la solution de monomères étant polymérisée pour obtenir un gel, le gel étant séché puis fractionné, **caractérisé en ce qu'**un moins l'un des paramètres influençant la polymérisation est modifié selon un modèle périodique.

7. Procédé selon la revendication 6, **caractérisé en ce qu'**au moins l'un des paramètres suivants est modifié :
- la concentration du/des monomère(s)
- la quantité de catalyseur
- la quantité du régulateur du poids molaire
- la valeur du pH de la solution de monomères et/ou
- la composition des monomères utilisés.

8. Procédé selon l'une des revendications 6 ou 7, **caractérisé en ce que** la polymérisation a lieu sur un support mobile.

9. Procédé selon l'une des revendications 6 à 8, **caractérisé en ce qu'**après le séchage la composition de polymères est pulvérisée, et le polymère en poudre est mélangé avec 0,05 à 3% en poids d'un composé pouvant réagir avec au moins deux groupes carboxyliques, chauffé à 150 - 250°C et réticulé superficiellement.

10. Utilisation de la composition de polymères selon l'une des revendications 1-5 comme agent d'absorption d'eau et de liquides aqueux.

11. Utilisation de la composition de polymères selon l'une des revendications 1-5 comme agent d'absorption dans des structures destinées à absorber des liquides corporels.

12. Utilisation de la composition de polymères selon l'une des revendications 1-5 comme composant absorbant de l'eau et des liquides aqueux dans des câbles électriques ou optiques, comme composant dans des matériaux d'emballage, comme agent d'amélioration du sol et pour la culture de plantes.

13. Utilisation de la composition de polymères selon l'une des revendications 1-5 comme agent d'absorption d'eau et de liquides aqueux dans des structures superficielles de préférence moussées.

14. Utilisation de la composition de polymères selon l'une des revendications 1-5 comme substance de support d'engrais ou d'autres agents actifs qui sont restitués pendant une période prolongée.
